(19) Europäisches Patentamt
European Patent Office
Office européen des brevets

(11) **EP 4 481 387 A1**

(12) **EUROPEAN PATENT APPLICATION**

(43) Date of publication:
**25.12.2024  Bulletin 2024/52**

(21) Application number: **23180131.7**

(22) Date of filing: **19.06.2023**

(51) International Patent Classification (IPC):
**G01N 33/543** *(2006.01)*     **A61B 10/00** *(2006.01)*

(52) Cooperative Patent Classification (CPC):
**G01N 33/54388; A61B 10/0012; A61B 10/007;**
A61B 2010/0006

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Withings**
**92130 Issy-Les-Moulineaux (FR)**

(72) Inventor: **Lefebvre, Laura**
**92130 Issy-les-Moulineaux (FR)**

(74) Representative: **Withings IP**
**2, rue Maurice Hartmann**
**92130 Issy-les-Moulineaux (FR)**

(54) **EXPANDED TEST STRIP**

(57)    The invention concerns a test strip (600) for detecting an analyte of interest present in a fluid. The test strip (600) is configured to receive a fluid sample and comprises a fluid pathway (602) for fluid of the fluid sample. The test strip (600) has a maximal expanse (E) along a main axis (X). A curvilinear length (L) of the fluid pathway (602) extends on a distance greater than twice the maximal expanse (E) of the test strip (600).

**FIG. 11**

## Description

## Field of the invention

The present invention relates to a test strip to detect an analyte of interest present in a fluid of a user. The fluid may be urine, blood, sweat for example. When the fluid is urine, the test strip may be designed to be arranged in a cartridge designed to be inserted in a station for urine analysis. The station may be installed on the surface of a toilet bowl. The cartridge mounted on the station will be referred to as an analysis device. The station comprises an analyzer, notably comprising a light source and a light sensor, to carry out an optical analysis on the test strip to detect the analyte of interest.

## Background

The urine of a user may be a source of useful information about a user. The analyte of interest may for example cancer markers for early detection of a new cancer or cancer relapse or may be for example Luteinizing hormone (LH) to detect an ovulation of a female user.

Lateral Flow test strips are widely used and known to detect analytes by immunochromatography. The concept of immunochromatography is a combination of chromatography (separation of components of a sample based on differences in their movement through a sorbent) and immunochemical reactions. Lateral Flow test strips are used commercially for the detection of pregnancy, for following up on ovulation, for covid tests, etc.

The principle of the assay is notably based on the ELISA (for "enzyme-linked immunosorbent assay") procedure. For example, when the analyte of interest is an antigen, antibodies are selected so they can attach to the antigens of interest, to immobilize them on a membrane. Conventionally, a test strip is a straight strip comprising a sample pad where the urine sample is dispensed, a conjugate pad comprising detection antibodies (conjugated to a colored or fluorescent element), a reaction membrane comprising immobilized capture antibodies and a wicking pad creating a capillary flow through the test strip.

When a urine sample is dispensed on the sample pad, the analyte is migrating from the sample pad to the wicking pad. The antigen of interest, if present in the urine sample, binds to the detection antibody of the conjugate pad to form a complex. Then, this complex migrates to the test line. The complex antigen/antibodies migrates to the reaction membrane on the membrane, where the complex binds to the immobilized capture antibodies. However, the detection antibodies alone do not bind to the immobilized capture antibody on the test line. Therefore, in an ELISA-sandwich configuration, if the antigen of interest is present in the urine sample, because of the colored or fluorescent element, a change of color of the reaction membrane may then be detected.

There is a need for implementing this assay in a urine analysis device as disclosed for example in document WO2021/175909. This document discloses a point-of-care device for urine analysis. The device is to be lodged in a toilet (more precisely on a surface of the toilet bowl) and collects samples of a urine stream before performing an optical analysis. The device comprises a station and a cartridge, which is also called a rotatable support, and which may be removed and replaced from the station. The cartridge contains urinary test strips, that is to say strips coated or impregnated with a reagent that reacts with urine.

The dimension of such a cartridge imposes to produce test strips with a reduced length, for example less than 2 cm, even less than 1.5 cm. This drastic reduction of the length of the test strip creates a big setback in sensitivity.

Indeed, the efficiency of the immunochemical reaction is decreased with the reduction of the length of the liquid pathway. According to the Lucas-Washburn equation, the speed of the fluid through the liquid pathway gradually decreases along the length of the liquid pathway:

$$L = \sqrt{\frac{\gamma r t \cos(\phi)}{2\eta}}$$

with L being the distance traveled by the liquid through the membrane ("liquid pathway"),

> t being the time needed to cover the distance L,
> $\gamma$ being the surface tension,
> $\eta$ being the dynamic viscosity,
> $\theta$ being the penetration angle of the liquid with the solid and
> r being the pore radius of the membrane.

In other words, the speed of urine is higher in the upstream part of the liquid pathway, near the sample pad, than in the downstream part of the liquid pathway, near the wicking pad.

The slower the urine flow is, the more time the molecules are in contact with each other, and therefore the reaction works better. On the contrary, if the speed of the urine is higher, the reaction has less time to happen, so the sensibility is lower. With a reduction of the length of the strip, the speed of the fluid decreases less along the fluid pathway, so the reaction has less time to happen on the membrane, and the intensity of the reaction membrane decreases.

This drastic reduction of the length of the test strip and therefore the reduction of the reaction of the lateral flow causes for some analytes that the intensity of the reaction membrane is too weak and too thin to be accurately detected by a urine analysis device.

## Summary of the disclosure

An aim of the disclosure is to provide a test strip designed to be arranged in a cartridge which enables accurate analytes detection to provide to an individual a regular, easy, and cost-effective analyte monitoring in a fluid.

To that end, the disclosure relates to a test strip for detecting an analyte of interest present in a fluid, wherein the test strip is configured to receive a fluid sample and comprises a fluid pathway for fluid of the fluid sample, wherein the test strip has a maximal expanse along a main axis, wherein a curvilinear length of the fluid pathway extends on a distance greater than twice the maximal expanse of the test strip.

Thanks to this test strip design, the maximal expanse of the test strip may be maintained lower than the height of a cartridge chamber while increasing drastically the length of the urine pathway. The maximal expanse may also be referred as a bulk dimension or an overall dimension. Therefore, the test strip according to the invention may be inserted in the chamber and avoids changing the design of the whole cartridge and analysis system, while enabling to provide a better intensity and sensibility for detection. In particular, the extended length enables to slow down further the fluid flowing through the test strip, and in particular through the reaction membrane. The slower the fluid flow is, the more time the molecules may be in contact with each other, and therefore the reaction may work better. Moreover, the extended length of the test strip enables to provide larger pads arranged on the test strips with more reagent, and therefore more possible reactions.

In one embodiment, the fluid is urine.

In one embodiment, the curvilinear length of the fluid pathway extends on a distance greater than three times the maximal expanse of the test strip.

In one embodiment, at each point of the fluid pathway a fluid flow defines a flow direction, wherein the fluid pathway comprises at least a change of flow direction of at least 90°, notably at least 135°.

In one embodiment, the fluid pathway comprises at least two straight portions at an angle which is non zero, in particular at least 90°.

In one embodiment, the fluid pathway comprises at least a change of flow direction of 180°.

In one embodiment, the fluid pathway comprises at least two straight portions which are parallel to each other but not aligned.

In one embodiment, the two portions form a U-turn for the fluid pathway.

In one embodiment, the fluid pathway comprises at least two changes of flow direction of at least 90°, notably at least 135°.

In one embodiment, the fluid pathway comprises three straight portions at an angle which is non zero.

In one embodiment, two straight portions following each other form between them an angle of at least 90°, notably at least 135°.

In one embodiment, the angles are sensibly 180°, so that the fluid pathway comprises at least three straight portions which are parallel to each other and not aligned.

In one embodiment, the fluid pathway comprises at least two changes of flow direction of 180°.

In one embodiment, the three straight portions form two U-turns for the fluid pathway.

In one embodiment, the fluid pathway comprises at least an inflection point.

In one embodiment, none of the straight portions are overlapping.

In one embodiment, two straight portions are at least partially overlapping.

In one embodiment, the maximal expanse is lower than 2cm, notably lower than 1,5cm (for example 1.2cm).

In one embodiment, a width of the fluid pathway is less than 5 mm, notably between 0.5 mm and 3 mm.

In one embodiment, the fluid pathway includes at least one connection portion.

In one embodiment, each connection portion is a straight portion or a curved portion.

In one embodiment, the fluid pathway comprises: a sample pad configured to receive the fluid sample; and a single wicking pad, configured to absorb fluid from the fluid sample on the sample pad, so that fluid flows from the sample pad to the single wicking pad.

In one embodiment, the fluid pathway further comprises: a conjugate pad between the sample pad and the wicking pad, a reaction membrane between the conjugate pad and the wicking pad.

In one embodiment, the test strip further comprises a backing on which the sample pad, the wicking pad, the conjugate pad and the reaction membrane are arranged.

In one embodiment, the fluid pathway comprises in order: a first straight portion, a first curved portion, a second straight portion, a second curved portion, a third straight portion.

In one embodiment, the sample pad is on the first straight portion.

In one embodiment, the conjugate pad is at least on the first curved portion.

In one embodiment, the reaction membrane is on the second straight portion.

In one embodiment, the wicking pad is at least on the second curved portion and/or on the third straight portion.

In one embodiment, each of the sample pad, wicking pad, and conjugate pad extends on a curvilinear length greater than the third, notably the half of the maximal expanse of the test strip.

In one embodiment, the analyte of interest is chosen between: an ion of interest, an antigen of interest, an antibody of interest.

In one embodiment, the detection being made by immunochromatography.

In one embodiment, the analyte of interest is chosen between: LH, hCG, FSH, progesterone, cancer markers, coronavirus.

In one embodiment, the conjugate pad comprises a predetermined amount of detection antibodies associated with the antigen of interest, wherein each antigen of interest is able to bind with one of the detection antibodies to form a complex, wherein the formed complexes and the free antibodies are able to flow towards the reaction membrane, wherein the reaction membrane comprises primary capture antibodies associated with the complex and fixed along a test line, wherein each formed complex is able to bind with one of the primary capture antibodies.

In one embodiment, the detection antibody is conjugated to a colored/fluorescent nanoparticle.

In one embodiment, the absorbance of the test line is measured by spectrophotometry after the fluid sample flow through the reaction membrane, a change of absorbance being representative of the presence of the antigen of interest.

In one embodiment, the color of the test line is determined with a camera after the fluid sample flow through the reaction membrane, a change of color being representative of the presence of the antigen of interest.

In one embodiment, the reaction membrane comprises secondary capture antibodies associated with the free detection antibodies and arranged along a control line, wherein each free detection antibody is able to bind with one of the secondary capture antibodies.

In one embodiment, the conjugate pad comprises a predetermined amount of detection antibodies associated with the antigen of interest, wherein each antigen of interest is able to bind with one of the detection antibodies to form a complex, wherein the formed complexes and the free antibodies are able to flow towards the reaction membrane, wherein the reaction membrane comprises a predetermined amount of reference antigens of the same type as the antigens of interest and fixed along a test line, wherein each free antibodies is able to bind with one of the reference antigens to form a reference complex.

In one embodiment, the detection antibody is conjugated to a colored/fluorescent nanoparticle.

In one embodiment, the absorbance of the test line is measured by spectrophotometry after the fluid sample flow through the reaction membrane, a change of absorbance being representative of the absence of the antigen of interest.

In one embodiment, the color of the test line is determined with a camera after the fluid sample flow through the reaction membrane, a change of color being representative of the absence of the antigen of interest.

The invention also concerns a cartridge for an optical fluid analysis device, the cartridge comprising at least one chamber wherein the at least one chamber comprises at least a test strip as described above.

In one embodiment, the at least one chamber comprises a single test strip, the single test strip comprising a single fluid pathway.

In one embodiment, the at least one chamber includes a plurality of chambers.

In one embodiment, the plurality of chambers is arranged next to one another in a right circular cylinder shape of at least 80% of a circle with a diameter comprised between 3 cm and 10 cm.

In one embodiment, at least one chamber has a maximum dimension, e.g., a height, which is less than 10% more than the maximal expanse of the test strip.

In one embodiment, the height of the chamber is lower than 2 cm, notably lower than 1,5 cm.

The invention also concerns a fluid optical analysis device comprising: a cartridge as described as above, a station, configured to be positioned on a wall of a toilet bowl, the station comprising: a case comprising a compartment in which the cartridge is at least partially received, an injector, configured to inject fluid on the test strip, an analyzer to analyze a change of property of the test strip due to fluid from the fluid sample.

In one embodiment, the compartment is an annular compartment in which the cartridge is at least partially received and is rotatably mounted around a rotation axis.

In one embodiment, the analyzer includes a light source and a light sensor, configured to emit and receive light.

In one embodiment, the light sensor is a camera able to detect a change of color of the test line and/or the control line.

In one embodiment, the light sensor is configured to measure the absorbance of the test line and/or control line.

The disclosure also relates to a test strip for detecting an analyte of interest present in fluid for a fluid optical analysis device cartridge, wherein the test strip is configured to receive a fluid sample and comprises a fluid pathway for fluid of the fluid sample, wherein the test strip has a maximal expanse along a main axis, wherein a curvilinear length of the fluid pathway extends on a distance greater than twice the maximal expanse of the test strip.

The disclosure also relates to a test strip for detecting an analyte of interest present in fluid, wherein the test strip is configured to receive a fluid sample and comprises a fluid pathway for fluid of the fluid sample, wherein at each point of the fluid pathway a fluid flow defines a flow direction, wherein the fluid pathway comprises at least a change of flow direction of at least 90°, notably at least 135°. The features disclosed above also apply.

The disclosure also relates to a test strip for detecting an analyte of interest present in fluid, wherein the test strip is configured to receive a fluid sample and comprises a fluid pathway for fluid of the fluid sample, wherein the fluid pathway forms at least a U-turn.

## Brief description of the Drawings

These features and advantages of the invention will appear more clearly upon reading the following description, provided solely as a non-limiting example, and done in reference to the appended drawings, in which:

- Figure 1 shows the overall setup of an analysis device for urine analysis according to an embodiment, as installed on a surface of a toilet bowl,

- Figure 2 shows an exploded view of an embodiment of an analysis device, in which the station and the cartridge are visible,

- Figure 3 shows a detailed view of a cartridge according to an embodiment,

- Figure 4 shows a sectional view of an embodiment of a cartridge and a station, at the location of an optical analyzer of the station,

- Figure 5 shows a definition of an angle between two vectors,

- Figures 6 to 11 show a top view of different test strips according to different embodiments of the invention,

- Figure 12 shows a comparison of the maximal expanse and the curvilinear length of a test strip according to the invention,

- Figure 13 shows a schematic sequence of a sandwich ELISA procedure,

- Figure 14 shows a schematic sequence of a competitive ELISA procedure,

- Figure 15 shows a graph representing the absorbance of the test line of a conventional test strip as a function of the wavelengths, and

- Figure 16 shows a graph representing the absorbance of the test line of a test strip according to the invention as a function of the wavelengths.

## Detailed description

The invention relates to test strip to detect an analyte of interest present in a fluid of a user. The fluid may be urine, blood, sweat. In the following, the description will be made with a fluid being urine, but it is noted that following applies to any fluid.

The present description introduces different examples of a cartridge usable with a station as disclosed in document WO2021/175909 and WO2021/175944, hereafter referred to as WO'909 and WO'944. Variations of the stations are presented in any of WO2023036805, WO2023036806, WO2023036808, WO2023036809, hereafter referred to as WO'80X.

The next paragraphs explain the overall principle of a device for urine analysis, but all the details of WO'909 and WO'933 (and also any of the above-mentioned French filings) are applicable.

## OVERALL DESCRIPTION OF THE STATION AND THE CARTRIDGE

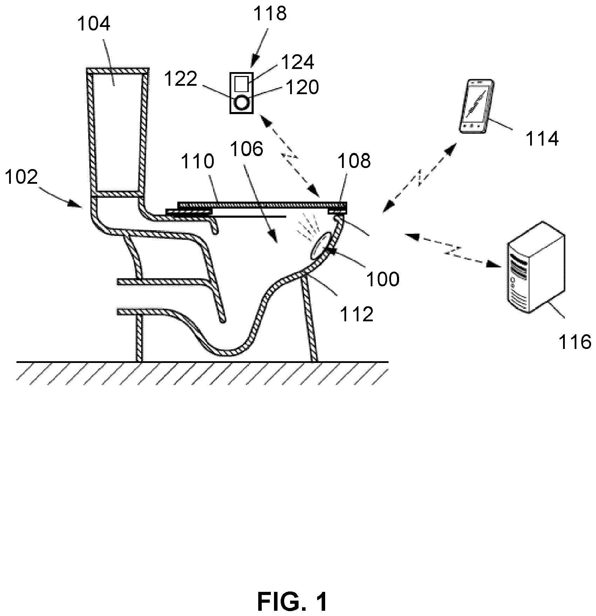

Figure 1 schematically illustrates an analysis device 100 (referred to as the "device 100") for urine analysis as set up in a toilet 102. Toilet 102 usually comprises a water tank 104, a bowl 106, a seat 108 and a seat cover 110. The analysis device 100 is arranged in a removable manner in the toilet 102. For example, the analysis device 100 may be easily removed from the toilet to replace a cartridge and then arranged again in the toilet 102. The analysis device 100 is arranged on an internal wall 112 of the bowl 106 of the toilet. The analysis device 100 is placed so that it is usually under a urine stream from a user, such that when a user urinates (typically in a seated position), urine contacts with the analysis device 100. The analysis device 100 may communicate remotely with a remote entity, such as smartphone 114 or a server 116.

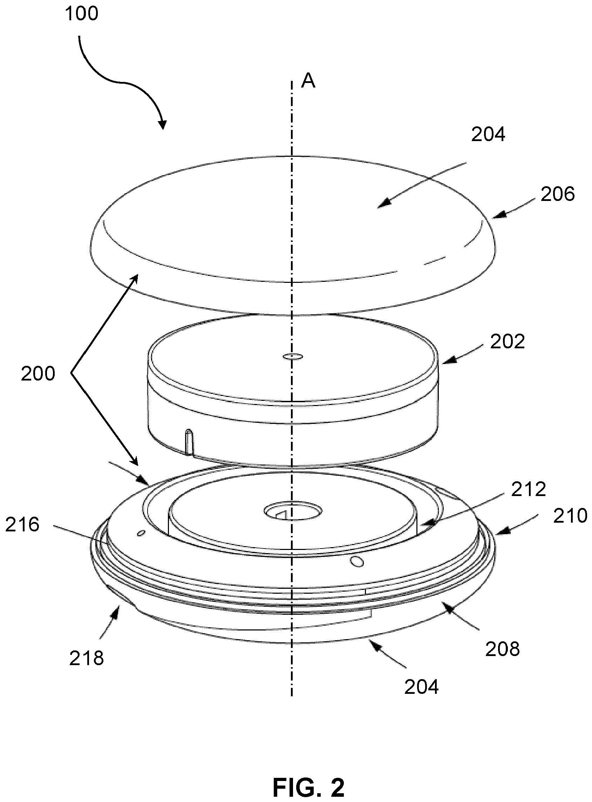

As illustrated in more detail on Figure 2, the analysis device 100 comprises a station 200 and a cartridge 202, mounted in a removable manner from the station 200. Station 200 may comprise a case 204 which may include two shells 206, 208. Case 204 lodges therein a urine testing assembly. Station 200 comprises an annular or ring-shaped compartment 212, located inside the case 204, arranged around a rotation axis A. The annular compartment 212 is configured to receive at least partially the cartridge 202 mounted in a rotatable manner around the rotation axis A (once in position in the annular compartment 212). The cartridge 202 comprises a plurality of test supports which each comprise at least one urine reagent, for instance a dry reagent, the plurality of test supports being arranged along a circle or a circular arc around the rotation axis A. In an embodiment, the test supports are test strips. The test supports may be enclosed, for example individually enclosed, in a chamber.

The annular compartment 212 typically extends around 360° and forms a groove configured to receive at least partially the cartridge 202.

Station 200 comprises a collection opening 218, located for example on shell 208. The collection opening 218 collects urine flowing on the surface of the case 204. A drain opening (not illustrated) is also included to drain the liquid out of the device 100.

The case 204 may have a diameter, in a direction perpendicular to the rotation axis A, comprising between 50 mm and 150 mm.

The test assembly may comprise a pump, an injector, and an analyzer. The pump sucks urine from the collection opening 218, then the injector injects the urine on one or more test supports of the cartridge and the analyzer obtains some values of properties (e.g., physical/chemical properties, such as the color) of the test supports after it has contacted the urine. In one embodiment, the analyzer is an optical analyzer configured to analyze optical properties of the test support. The injector and the cartridge may move relatively to each other so that the injector can open (e.g., pierce) the chamber, for example with a needle or needle-like device.

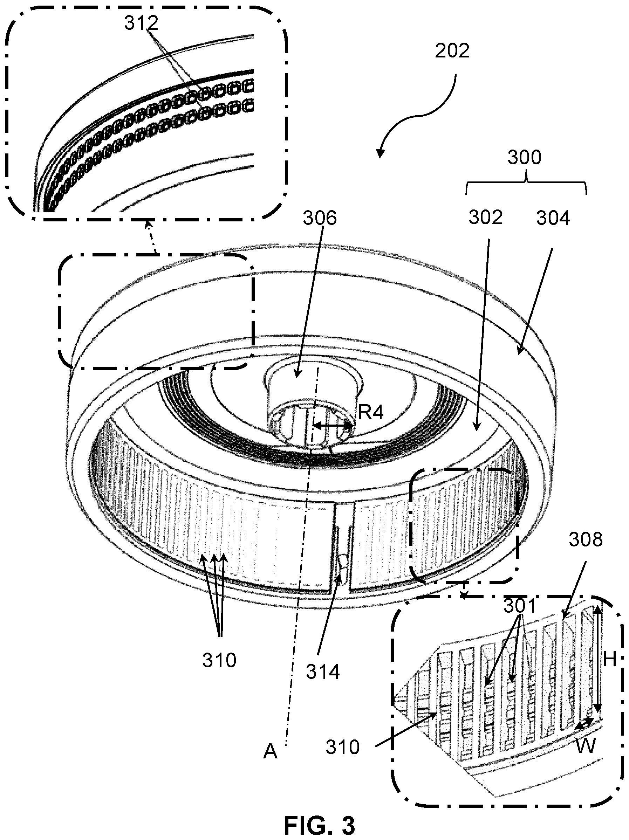

Figure 3 shows an exploded view of the cartridge 202. The cartridge 202 comprises at least a test support 301, notably several test supports 301 configured to receive urine from the injector. Each test support 301 contains a urine reagent that reacts in a specific way when in contact with urine. The cartridge 202 comprises a rotatable support 300, configured to be driven in rotation by the station 200. In normal use of the cartridge 202 and the device 100, the test supports 301 remain attached to the rotatable support and do not move with respect to the latter.

In an embodiment, the rotatable support 300 has a right circular cylinder shape of at least 80% of a hollow cylinder shape extending annularly around an axis which is, when the cartridge 202 is mounted in the station 200, the rotation axis A. Each test support 301 may be a test strip. The rotatable support 300 may comprise an annular portion 302 and a cylindrical portion 304, which extends from an outermost radial extremity of the annular portion 302. The cylindrical portion 304, when in use, is lodged inside the annular chamber 212. The test supports 301 are positioned along the cylindrical portion 304, so that they can be selectively and/or successively scrolled in front of the injector and the analyzer. For instance, the test supports 301 are part of a holder 308, which comprises several chambers 310, separated from each other along a perimeter around the axis A. At least a test strip is received in a chamber 310. Advantageously, a single test strip is received in a chamber 310. In particular, each chamber 310 comprises a single test strip.

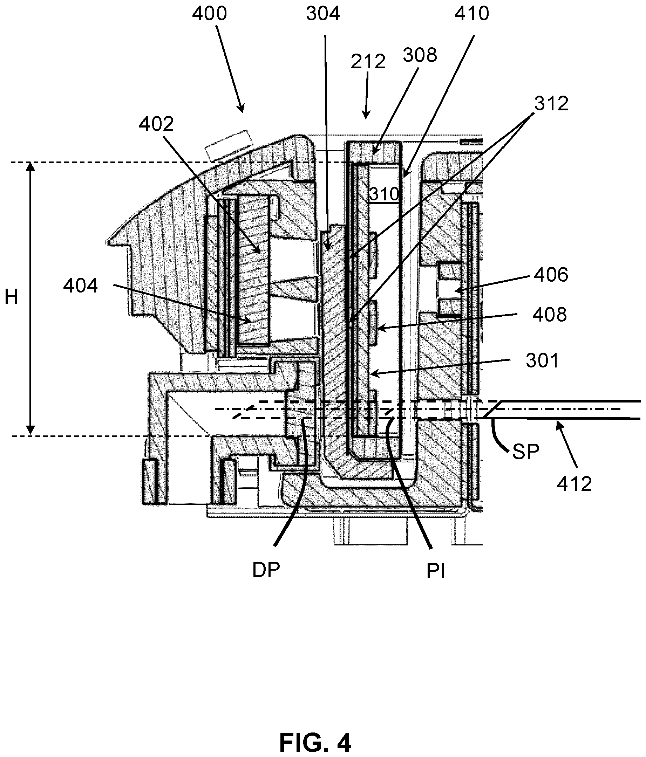

The plurality of chambers 310 are arranged next to one another in a right circular cylinder shape of at least 80% of a circle. To allow light to go through, the holder 308 includes at least one aperture 312 per chamber 310 (represented in the upper left zoom where the rotatable support is shown as transparent). The chambers 310 are all at an equal distance of the rotation axis A, so that the injector can selectively inject urine after the desired chamber is positioned at a desired location facing the injector. The injector may translate towards the chamber 310 and pierce a lid closing the chamber 310 (visible on Figure 4). A drain opening 314 is provided in the rotatable support 300 to allow evacuating urine from the injector to the outside of the device 100.

Each chamber 310 may have a maximum dimension, e.g., a height H, which is less than 10% more than the maximal expanse of the test support, as it will be explained below. In particular, each chamber 310 may have a height along the rotation axis A lower than 2 cm, notably lower than 1,5 cm.

Each chamber 310 may have a transversal width W, orthogonally to the rotation axis A, comprised between 1 mm and 6 mm.

The annular portion 302 of the rotatable support 300 remains outside of the annular chamber 212 to strengthen the cylindrical portion and/or to drive in rotation the cartridge 202. To that end, the annular portion 302 may include a mechanical coupling 306, which cooperates with a shaft of the station 200.

The dimensions relative to the cartridge 202 are disclosed in WO'909, WO'933, and WO'80X. The maximum dimension of the device 100 transversal to the rotational axis A is less than 15 cm, even less than 10 cm. The maximum dimension of the device along the rotation axis A is less than 5 cm.

Figure 4 shows in more detail the interaction between the cartridge 202 and the station 200 when or after the injector is activated. The analyzer 400 comprises at least one light source 402, 404 (e.g., two) and at least one optical sensor 406. Light travels from the light source 402, 404 to the optical sensor 406 while passing through the cartridge 202 and in particular the cylindrical portion 304, the aperture 312 of the holder 308, the test support 301 and thus the reagent 408.

In one embodiment, the analyzer 400 is configured to measure the absorbance of a part of the test supports 301 (notably the test line and/or control line of a test strip as it will be explained below). The absorbance is detected by the light source (for example a led) which may pass light through the strip, and the optical sensor which receives the spectrum with about ten wavelengths.

In a variant, the light sensor is a camera able to detect a change of color, notably a change of intensity of color, of a part of the test supports 301 (notably the test line and/or control line of a test strip as it will be explained below). The camera may detect a color in RGB values for example.

The injector includes an injection end 412 (for example a needle), which can be moved between several positions, which are represented in dash lines in Figure 4. In a standby position SP, the injection end 412 is outside the cartridge 202 (in an innermost position), so that the cartridge 202 can rotate freely in the annular housing 212; in an injection position IP, the injection end 412 has pierced the lid 410 to access the inside of the chamber 310 and may inject some urine on the test support 301; in the drain position DP, the injection end 412 is able to evacuate urine through the drain opening 314 of the rotatable support 300.

In position SP, the injector is located radially inward the annular chamber. This allows to maximize the radius of the annular chamber while minimizing the size of the station 200.

## Shape of the test strip

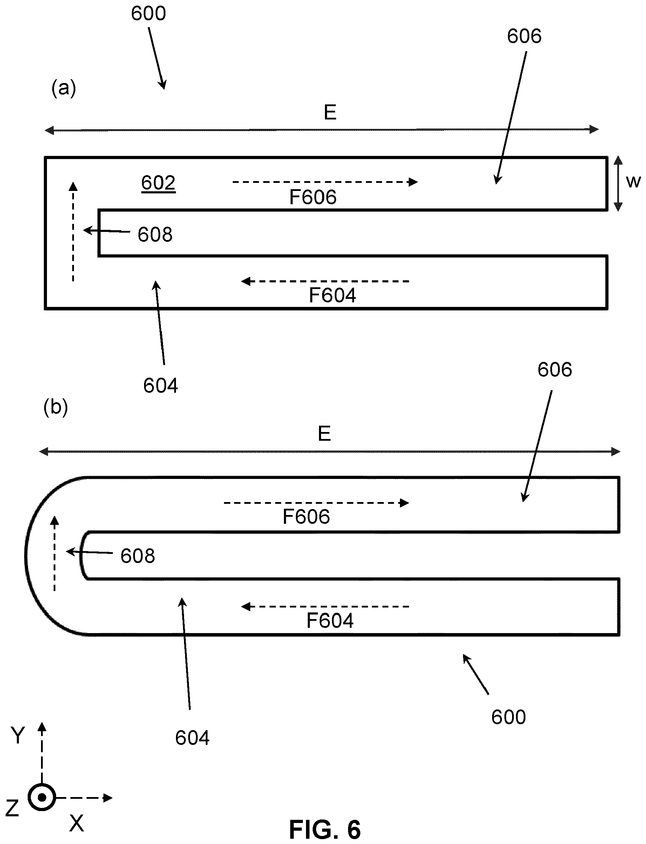

The test support 301 may be a test strip 600. The test strip 600 is configured to receive a urine sample and to react or interact with an analyte of interest present in urine of the urine sample. Fig. 6 to 10 shows various embodiments of such test strip 600.

The test strip 600 comprises a urine pathway 602 for a urine flow of the urine sample. In particular, the test strip 600 comprises a single pathway 602. The urine pathway 602 is the path along which the urine is flowing through the test strip 600. Urine pathway 602 has a direction, defined by the elements composing said pathway and configured to steer urine in one direction. In one embodiment, the urine pathway is unidirectional.

The urine pathway 602 mainly extends in a main plane XY. In other words, the overall width of the test strip 600 along an orthogonal axis Z to this plane XY is less, for example at least five times less, than the overall dimensions in the main plane XY. In use in the cartridge 202, the orthogonal axis roughly corresponds to a radial direction of the circular cylinder shape of the rotatable support 300.

At each point of the urine pathway 602, the urine flow defines a general flow direction F. The general flow is defined by the global flow of urine, being acknowledge that some local flows may occur in different directions. On the figures, the flow direction F is represented by a vector, referenced F, at each point of the urine pathway 602. When a pathway is straight in one direction, the flow direction is also in the same direction and the vector is also in the same direction.

The urine pathway 602 may present a width w, transversal to the flow direction, inferior to 5 mm, notably between 0.5 mm and 3 mm.

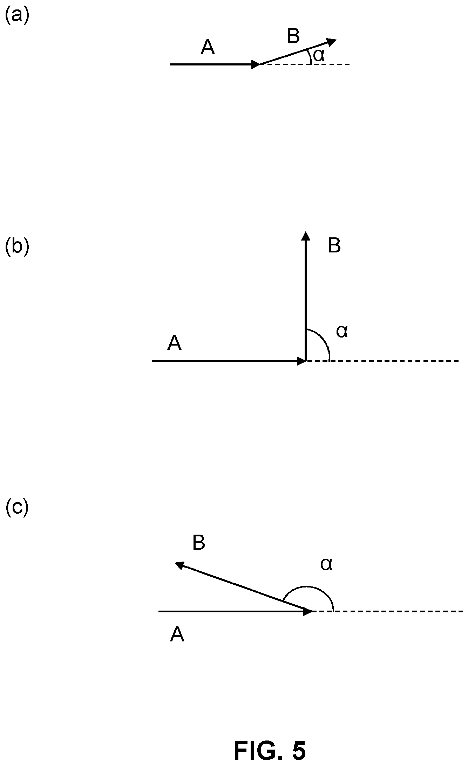

Figure 5 shows the definition of an angle between two vectors. A vector has an origin and a direction. The mathematic formula defines the angle $\alpha$ between two vectors A and B, by the formula:

$$cos\ \alpha\ =\ \frac{A.B}{|A|\,|B|}\ ,$$

where A.B is a scalar product and |x| is the magnitude. The angle between two vectors is defined between 0° and 180° included. When the two vectors are aligned, the angle is equal to 0° and when the two vectors are opposite the angle is 180°.

Three examples are illustrated on Figure 5. In example (a), the two vectors A and B are sensibly oriented in the same directions and define between them an angle $\alpha$ lower than 45°. In example (b), the two vectors A and B are sensibly orthogonal and define between them an angle $\alpha$ of 90°. In example (c), the two vectors A and B are sensibly oriented in opposite directions and define between them an angle $\alpha$ greater than 135°.

As visible on the Figures, the urine pathway 602 comprises at least a change of flow direction F of at least 90°, and in particular at least 135°. In other words, at least two vectors at two different points of the urine pathway 602 have an angle of at least 90° between them, and in particular at least 135°.

The urine pathway 602 may comprise at least two straight portions 604, 606. The straight portions 604, 606 are not necessarily adjacent, but may have another portion therebetween. Each straight portion is a portion in which the urine is flowing sensibly along a single direction, without a curve or bend. The two straight portions comprise a first straight portion 604 and a second straight portion 606. Each straight portion 604, 606 may be associated to a single vector F604, F606, whose direction depends on the urine flow configuration of the straight portion of the urine pathway 602. For example, on Figure 6, embodiments (a) and (b), the two vectors F604, F606 have opposite directions.

The urine pathway 602 may further include at least one connection portion 608 between the two straight portions 604, 606. It is therefore understood that, in disclosure, the two straight portions are not necessarily defined as consecutive. The connection portion 608 may be a straight portion as represented on embodiment (a) of Figure 6 or a curved portion as represented on embodiment (b) of Figure 6. Each curved portion is a portion in which the urine is flowing along a curve to change direction.

In the embodiment illustrated on Figure 6, the test strip 600 comprises a single connection portion 608 between the first straight portion 604 and the second straight portion 606.

As visible on Figure 6, the two straight portions 604, 606 (or their associated vectors) form an angle of 180°, which is non zero. Said angle is at least 90°, notably at least 135°.

As illustrated on Figure 6, in an embodiment, said angle is sensibly 180°, so that the urine pathway 602 comprises at least two straight portions 604, 606 which are parallel to each other but not aligned. In other words, the urine pathway 602 forms at least a U-turn.

In this embodiment, none of the straight portions 604, 606 are overlapping, i.e. in the Z direction. In other words, none of the straight portions covers, even partially, another straight portion. All the straight portions extend in the main plan XY.

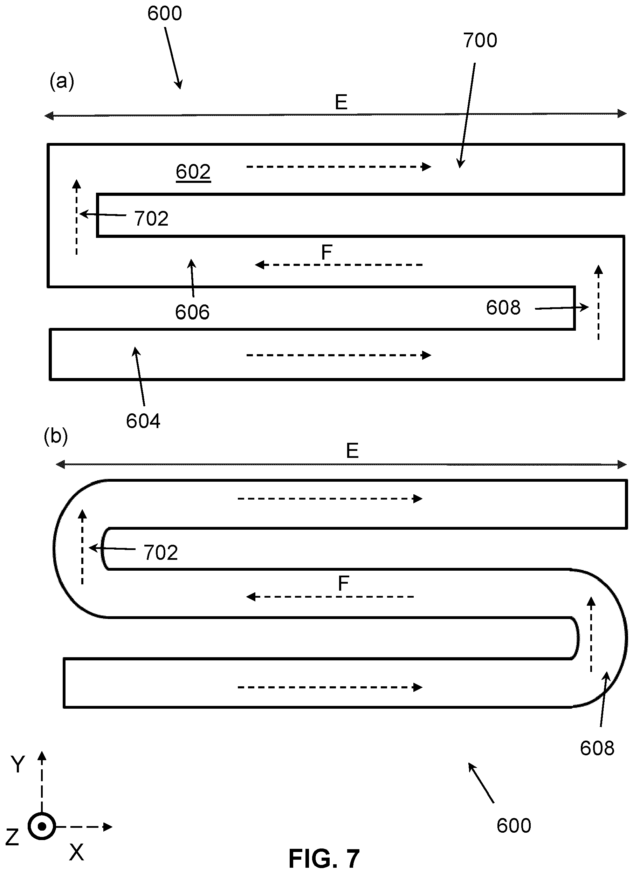

In a variant illustrated on Figure 7, the urine pathway 602 may comprise three straight portions 604, 606, 700 at an angle which is non zero. In particular, the first straight portion 604 and the second portion 606 form a first angle and the second straight portion 606 and the third portion 700 form a second angle. The urine pathway 602 may further comprise two connection portions 608 (between the first straight portion 604 and the second straight portion 606), 702 (between the second straight portion 606 and the third straight portion 700). Each connection portion may be a straight portion as represented on embodiment (a) of Figure 7 or a curved portion as represented on embodiment (b) of Figure 7. The urine pathway 602 comprises thus, in the following order: the first straight portion 604, the first connection portion 608, the second straight portion 606, the second connection portion 702 and the third straight portion 700. Two straight portions 604, 606 following each other, directly or not, form between them an angle of at least 90°, notably at least 135°. By "following portions", it is understood that the portions are next to each other on the urine pathway, but with eventually a curved portion between them. Said angles are sensibly equal to 180°, so that the urine pathway 602 comprises at least three straight portions which are parallel to each other and not aligned. In other words, the urine pathway 602 forms two consecutive U-turns.

In this embodiment, none of the straight portions are overlapping. All the straight portions extend in the main plan XY.

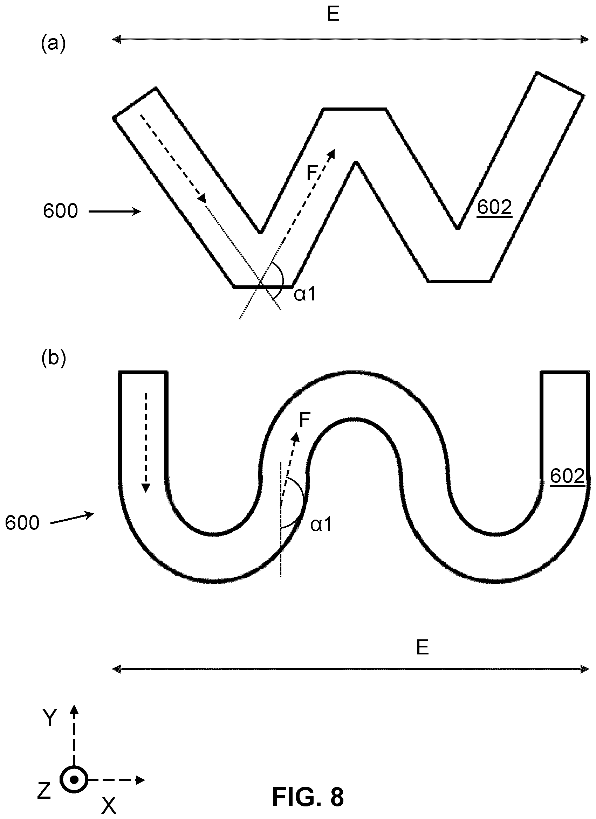

In a variant illustrated on Figure 8 (a), the urine pathway 602 may comprise at least two straight portions, for example four or five straight portions, following directly each other.

In a variant illustrated on Figure 8 (b), the urine pathway 602 may comprise at least two curved portions, for example three curved portions, following directly each other. The sign of curvature changes between each curved portions. In other words, an inflection point is present between two curved portions.

In this embodiment, none of the straight portions are overlapping. All the straight portions extend in the main plan XY.

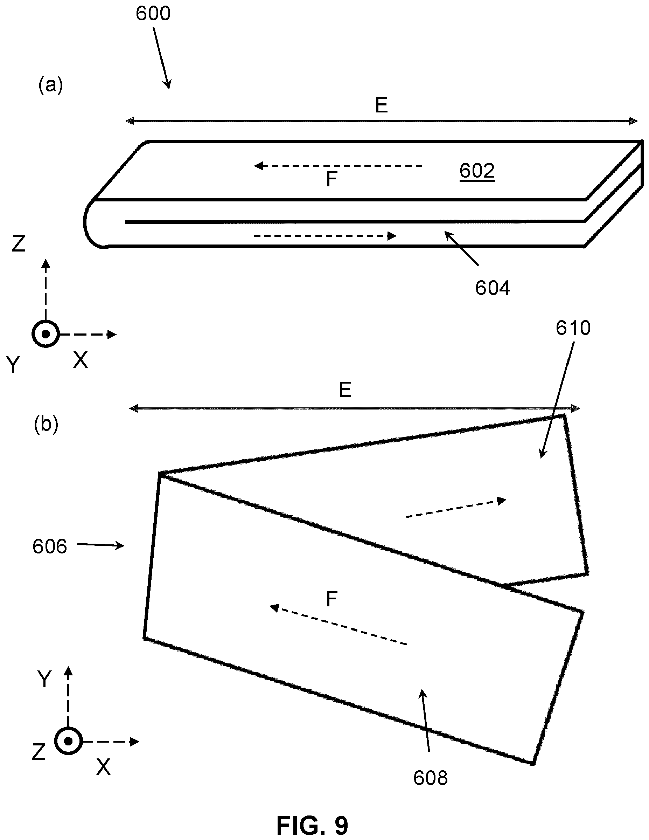

In a variant represented on Figure 9, two straight portions of the test strip 600 are at least partially overlapping.

In the embodiment (a) illustrated on Figure 9, strip 600 has two straight portions 602, 604 may completely overlap. In other words, one of the straight portions completely covers the other one. In particular, the two straight portions are stacked one above the other along the Z direction.

In the variant illustrated on embodiment (b) of Figure 9, strip 606 has two straight portions 608, 610 which are only partially overlapping, i.e., at least a part of a straight portion 608, 610 is not covered by the other.

In the embodiments illustrated on Figure 1 to 9, the different portions are transversally away from each other. In other words, there is no contact between the lateral sides of each portion.

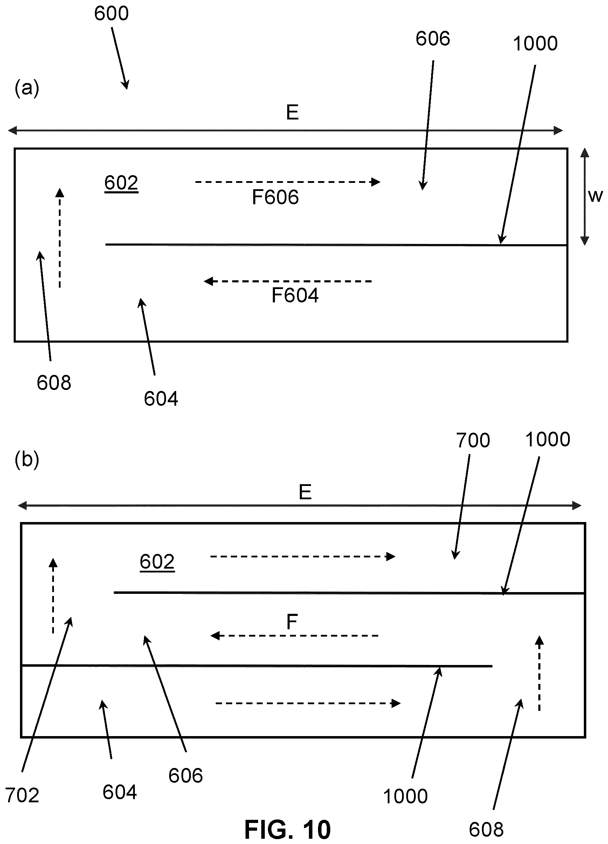

In a variant illustrated on Figure 10, the test strip is made of a single sheet and the different portions are fluidically separated by a wall 1000. The wall 1000 is configured to direct the flow and avoid a fluidic bypass. The wall 1000 is made of a hydrophobic material, for example wax. The wax may be applied in liquid form and dries to form the wall 1000.

## Curvilinear length of the test strip

As visible on the Figures, the test strip 600 has a maximal expanse E along a main axis, for example axis X. The main axis X extends in the main plan XY. The maximal expanse E is the greater length of the test strip 600 along a straight direction. The maximal expanse E may be also referred as overall dimension, or bulk dimension. For example, the maximal expanse of a parallelepiped is its length, the maximal expanse of a U-turn is the length of one of the branches, the maximal expanse of a circle is its diameter, etc.

The maximal expanse E of the test strip 600 may be lower than 2 cm, notably lower than 1.5 cm. This maximal expanse E enables the test strip 600 to be inserted in a chamber 310 of a cartridge 202. In particular, the height of a chamber 310 of the cartridge is less than 10% more than the maximal expanse E of the test strip 600.

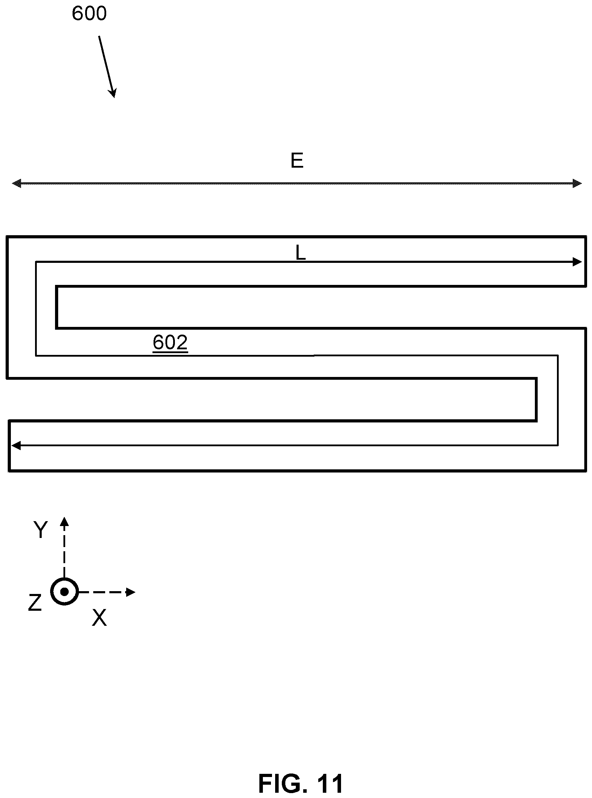

According to the invention, as represented on Figure 11, the curvilinear length L of the urine pathway 602 extends on a distance greater than twice the maximal expanse E of the test strip. Advantageously, the curvilinear length L of the urine pathway 602 extends on a distance greater than three times the maximal expanse E of the test strip 600. The curvilinear length is the distance between the two extremities of the urine pathway following all the straight and curved portions. For example, the curvilinear length of a U-turn test strip as illustrated on Figure 6 (a) is the sum of the length of the first straight portion 604, the length of the connection portion 608 and the length of the second straight portion 606.

With a conventional straight test strip, the length of the urine pathway would be less than, or at best equal to, the maximal expanse of the test strip. As explained above, this would lead to a big setback in sensitivity as the urine would flow too quickly along the urine pathway, and in particular through the reaction membrane. With a test strip designed according to the invention, the inventors have achieved to decorrelate the maximal expanse of the test strip and the length of the urine pathway. Thanks to the invention, the maximal expanse of the test strip may be maintained lower than the height of a cartridge chamber while increasing drastically the length of the urine pathway. Therefore, the test strip may be inserted in the chamber and avoids changing the design of the whole cartridge and urine analysis system, while enabling a better intensity and sensibility for detection, as it will be shown more in detail below. In particular, the extend length enables to slow down further the urine flowing through the test strip, and in particular through the reaction membrane. The slower the urine flow is, the more time the molecules may be in contact with each other, and therefore the reaction may work better. Moreover, the extended length of the test strip enables to provide larger pads arranged on the test strips with more reagent, and therefore more possible reactions.

## Composition of the test strip

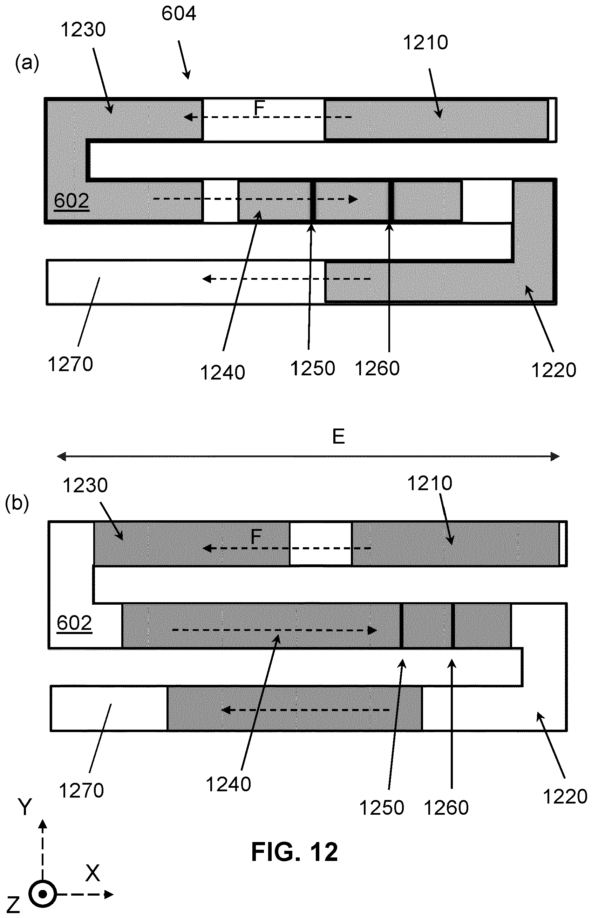

In reference to Figure 12, the urine pathway 602 may comprise a sample pad 1110, a single wicking pad 1120, a conjugate pad 1130 and a reaction membrane 1140.

In the embodiment illustrated on Figure 12, the different pads do not overlap. In a variant not shown, at least two pads may be in contact, and may even overlap, to enable a better fluid transition between them.

The sample pad 1110 is arranged, by definition, at an extremity of the urine pathway 602. The sample pad 1110 is configured to receive a urine sample. In particular, the sample pad is configured to face the injector end 412 when the test strip 600 is arranged in a chamber 310 of the cartridge 202 and placed inside the analysis device 100. The sample pad 700 is configured to receive the urine injected by the injector on the test strip 600.

The urine sample may comprise an analyte of interest to be detected. The analyte of interest may be chosen between: an ion of interest, an antigen of interest, an antibody of interest. In particular, the analyte of interest may be chosen between: LH, hCG, FSH, progesterone, cancer markers, coronavirus, etc. As it will be explained in detail above, the detection of the analyte of interest may be made by immunochromatography.

The wicking pad 1120 is arranged, by definition, at the other extremity of the urine pathway 602, opposite to the sample pad 1110. The wicking pad 1120 is configured to absorb urine from the urine sample on the sample pad 1110, so that urine flows from the sample pad 1110 to the single wicking pad 1120 along the urine pathway 602.

The conjugate pad 1130 and the reaction membrane 1140 are arranged between the sample pad 1110 and the wicking pad 1120. The reaction membrane 1140 is following the conjugate pad 1130 along the urine flow of the urine pathway.

The conjugate pad 1130 may have a length between 2 mm and 15 mm along the urine pathway. In particular, the conjugate pad 1130 may have a length longer than 25% of the maximal expanse E, notably more than 30%. As a comparison, in a conventional test strip, the conjugate pad extends on approximately 15% of the length of the test strip.

The reaction membrane 1140 may have a length between 4 mm and 15 mm along the urine pathway. In particular, the conjugate pad 1130 may have a length longer than 40% of the maximal expanse E, notably more than 60%.

The reaction membrane 1140 may comprise at least a test line 1150 and a single control line 1160. In the embodiment illustrated on Figure 12, the reaction membrane 1140 comprises a single test line 1150. In a variant, the reaction membrane 1140 may comprise several test lines 1150. Each test line 1150 may be associated to a different analyte of interest to detect. The test strip may therefore be configured to detect a plurality of analytes of interest. In complement or in a variant, at least two test lines may be associated to the same analyte of interest. The plurality of test lines enables a quantitative detection of the analyte of interest, each test line being triggered at a different concentration of the analyte. As visible on the Figures, the or each test line 1150 is arranged before the control line 1150 along the urine flow of the urine pathway 602.

The test strip 600 may further comprise a backing 1170 on which the sample pad 1110, the wicking pad 1120, the conjugate pad 1130 and the reaction membrane 1140 are arranged.

In the embodiment represented on Figure 9, the test strip is folded so that the backing 1170 is in the inside of fold and the pads are on the outside of the fold. so that the different pads do not touch each other.

In reference to Figure 12, the sample pad 1110 may be arranged on the first straight portion 604, at an extremity of the urine pathway 602.

The conjugate pad 1130 may be arranged on the first straight portion 604 as illustrated in the embodiment (b) of Figure 12. In a variant illustrated in the embodiment (a) of Figure 12, the conjugate pad 1130 is arranged at least on the first connection portion 608, and eventually on the first straight portion 604 and the second straight portion 606.

As illustrated in the embodiment (b) of Figure 12, the reaction membrane 1140 is arranged preferably on the second straight portion 606. In a preferred embodiment, the reaction membrane 1140 covers a majority of the second straight portion 606, for example at least 75% of the second straight portion 606. The longer the reaction membrane 1140 is, the more the urine will be slowed while flowing through it. Advantageously, the test line 1150 and the control line 1160 are arranged in the downstream part of the reaction membrane 1140, for example in the last 25% of the reaction membrane 1140, so as to enable the urine to be slowed significantly when reaching the lines 1150, 1160.

As illustrated in the embodiment (b) of Figure 12, the wicking pad 1120 may be arranged on the third straight portion 700. In a variant, illustrated in the embodiment (a) of Figure 12, the wicking pad 1120 may be at least arranged on the second connection portion 702 and eventually on the second straight portion 606 and the third straight portion 700.

However, the different pads may be arranged in any possible arrangement along the urine pathway 602.

**Sandwich ELISA procedure**

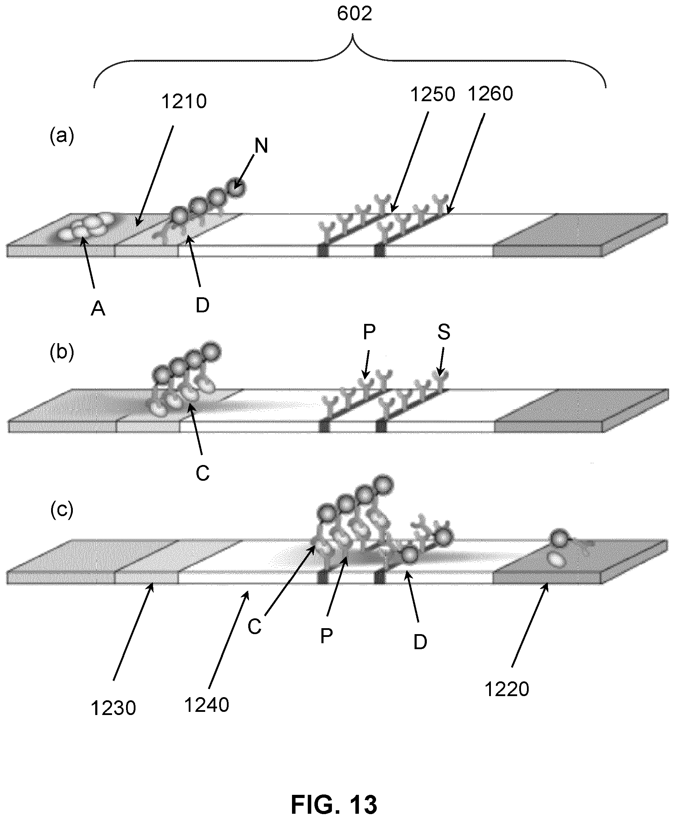

In reference to Figure 13, a first detection embodiment of the analyte of interest made by immunochromatography will now be described, called "Sandwich ELISA procedure". In Figure 13, the urine pathway 602 is represented in a straight way for clarity issues. However, the following explanation applies in a similar way for a curved urine pathway.

The analyte of interest is in this embodiment an antigen of interest.

The sample pad 1110 is configured to receive a urine sample comprising the analyte of interest A.

The conjugate pad 1130 may comprise a predetermined amount of detection antibodies D associated with the antigen of interest. Each antigen of interest A is able to bind with one of the detection antibodies D to form a complex C. The formed complexes C and the free antibodies D are able to flow towards the reaction membrane 1140.

The reaction membrane 1140 comprises primary capture antibodies P associated with the complex and fixed along the test line 1150. Each formed complex C is able to bind with one of the primary capture antibodies P.

Each detection antibody is conjugated to a colored or fluorescent nanoparticle N.

In an embodiment, the absorbance of the test line 1150 is measured by spectrophotometry after the urine sample has flown through the reaction membrane 1140. A change of absorbance of the test line 1150 is representative of the presence of the antigen of interest in the urine sample.

In a variant, the color of the test line 1150 is determined with a camera after the urine sample has flown through the reaction membrane 1140. A change of color of the test line 1150 is representative of the presence of the antigen of interest in the urine sample.

The reaction membrane 1140 may comprise secondary capture antibodies S associated with the free detection antibodies and arranged along the control line 1160. Each free detection antibody D is able to bind with one of the secondary capture antibodies S.

In operation, a urine sample comprising the antigens of interest A to detect is injected on the sample pad 1110 (step (a) on Figure 13). Primary capture antibodies P, directed against the antigens of interest A are immobilized on the test line 1150. If the antigen of interest is present, it binds to the detection antibody D (step (b) on Figure 13), then to the primary capture antibody P (step (c) on Figure 13), while migrating along the strip. The detection antibodies D is conjugated to a colored nanoparticle. Therefore, the antigen A is in sandwich between the couple of antibodies D, P, and the signal is visible thanks to the nanoparticles. If the antigen is not present in the analyte, then no signal is visible.

In a variant, the analyte of interest is an antibody of interest. In this case, the conjugate pad 1130 may comprise a predetermined amount of detection antigens associated with the antibody of interest and the reaction membrane 1140 comprises primary and secondary capture antigens.

## Competitive ELISA procedure

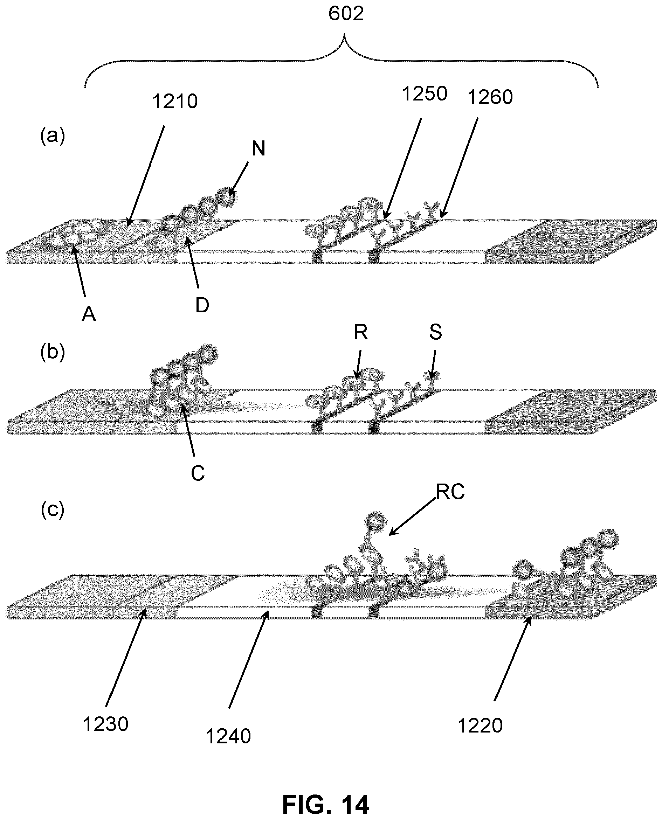

In reference to Figure 14, a second detection embodiment of the analyte of interest made by immunochromatography will now be described, called "Competitive ELISA procedure". In Figure 14, the urine pathway 602 is represented in a straight way for clarity issues. However, the following explanation applies in a similar way for a curved urine pathway.

The sample pad 1110 is configured to receive a urine sample comprising the analyte of interest A.

The conjugate pad 1130 comprises a predetermined amount of detection antibodies D associated with the antigen of interest. Each antigen of interest A is able to bind with one of the detection antibodies D to form a complex C. The formed complexes C and the free detection antibodies D are able to flow towards the reaction membrane 1140.

The reaction membrane 1140 comprises a predetermined amount of reference antigens R of the same type as the antigens of interest A and fixed along the test line 1150. Each free antibodies D is able to bind with one of the reference antigens R to form a reference complex RC.

Each detection antibody D is conjugated to a colored or fluorescent nanoparticle.

In an embodiment, the absorbance of the test line 1150 is measured by spectrophotometry after the urine sample has flown through the reaction membrane 1140. A change of absorbance of the test line 1150 is representative of the absence of the antigen of interest A.

In a variant, the color of the test line 1150 is determined with a camera after the urine sample has flown through the reaction membrane 1140. A change of color is representative of the absence of the antigen of interest A.

In operation, a urine sample comprising the antigens of interest A to detect is injected on the sample pad 1110 (step (a) on Figure 14). The reference antigen R, of the same type as the antigens of interest A, are fixed on the test line 1150. If the antigen of interest is in the urine sample, then it binds to the detection antibody D (step (b) on Figure 14). While migrating along the urine pathway 602, the detection antibody D is already bound to an antigen A, so it can't be bound to another antigen of reference R. Therefore, any change of color on the test line 1150 is detected. If the antigen of interest is not in the urine sample, the fixation site of the detection antibody D is free. Therefore, the detection antibody D binds to the immobilized antigen of reference R. A change of color is then visible on the test line 1150.

In a variant, the analyte of interest is an antibody of interest. In this case, the conjugate pad 1130 may comprise a predetermined amount of detection antigens associated with the antibody of interest and the reaction membrane 1140 comprises reference antibody and secondary capture antigens.

## Comparative tests

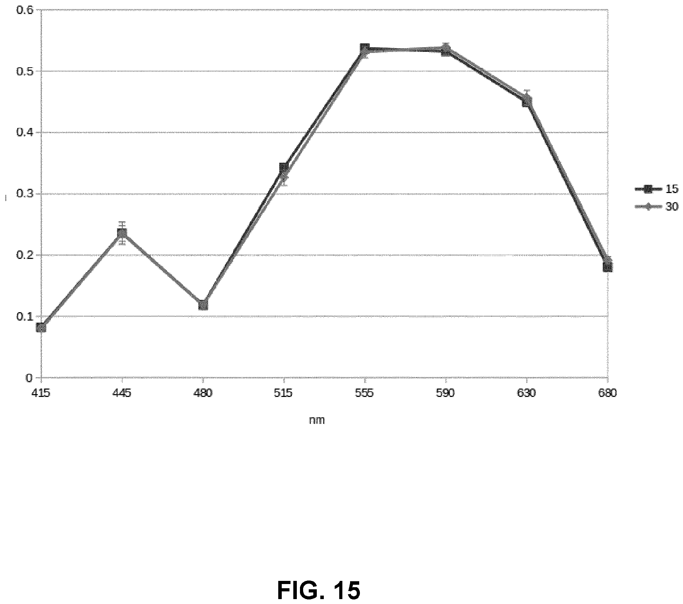

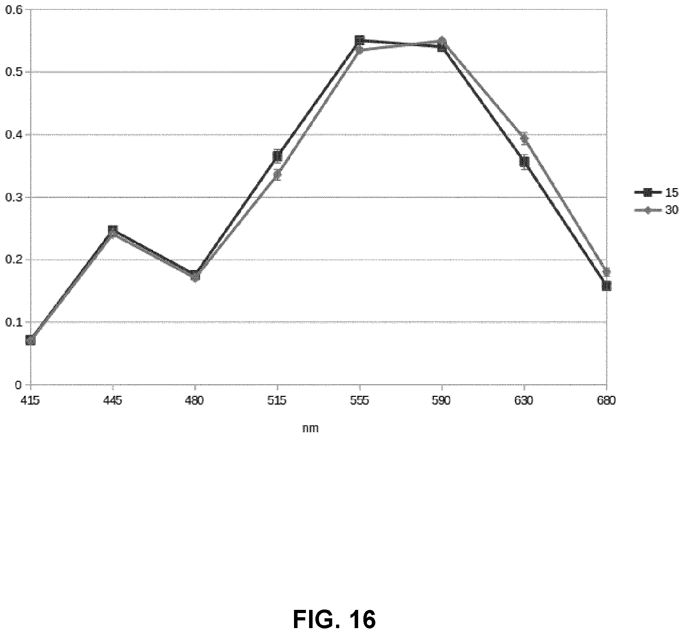

Figures 15 and 16 represent tests carried out by the inventors using a conventional test strip and a test strip 600 according to the invention.

The conventional test strip is a straight test strip of 1.2 cm long.

The test strip 600 according to the invention is a test strip similar to the one represented on Figure 12, presenting two U-turns and a maximal expense E of 1.2 cm.

The analyte of interest to detect is LH hormone (ovulation hormone). The threshold is usually set at 30 mIU/ml (milli-international unit per milliliter).

Therefore, for each test strip, two measures have been carried out at two different concentrations, one under the threshold at 15 mIU/ml (baseline, negative for ovulation) and one at the threshold of 30 mIU/ml (threshold, positive for ovulation).

Three strips of each type and for each concentration were evaluated with the analysis device 100. Each test strip was placed in the cartridge 202 and measured, twice.

The absorbance is measured, and the spectra is normalized by the following formula:

$$\lambda / \sqrt{\Sigma(\lambda^2)}$$

Then, the mean and the standard deviation for each concentration were calculated and plotted on the charts represented on Figures 15 and 16.

Referring to Figure 15, for the conventional test strip, the error bars (standard deviation) are overlapping. It is therefore not possible to distinguish the results between 15 mIU/ml and 30 mIU/ml.

Referring to Figure 16, for the test strip 600 according to the invention, the error bars (standard deviation) are not overlapping at 515 nm and 630 nm. It is therefore possible to distinguish the results between 15 mIU/ml and 30 mIU/ml.

The test strip according to the invention enables the intensity of the test line to be increased significantly. The invention enables to increase the resolution between two different concentrations (i.e. two lines with a different intensity level), while keeping the small maximal expanse required for the cartridge.

**Claims**

1. A test strip (600) for detecting an analyte of interest present in a fluid,

   wherein the test strip (600) is configured to receive a fluid sample and comprises a fluid pathway (602) for fluid of the fluid sample, wherein the test strip (600) has a maximal expanse (E) along a main axis (X), wherein a curvilinear length (L) of the fluid pathway (602) extends on a distance greater than twice the maximal expanse (E) of the test strip (600).

2. The test strip (600) according to claim 1, wherein the curvilinear length (L) of the fluid pathway (602) extends on a distance greater than three times the maximal expanse (E) of the test strip.

3. The test strip (600) according to claims 1 or 2, wherein at each point of the fluid pathway (602) a fluid flow defines a flow direction (F), wherein the fluid pathway (602) comprises at least a change of flow direction (F) of at least 90°, notably at least 135°.

4. The test strip (600) according to claim 3, wherein the fluid pathway (602) comprises at least a change of flow direction (F) of 180°.

5. The test strip (600) according to claims 3 or 4, wherein the fluid pathway (602) comprises at least two changes of flow direction (F) of at least 90°, notably at least 135°.

6. The test strip (600) according to claim 5, wherein the fluid pathway (602) comprises at least two changes of flow direction (F) of 180°.

7. The test strip (600) according to any of claims 1 to 6, wherein the fluid pathway (602) comprises at least an inflection point.

8. The test strip (600) according to any of claims 1 to 7, wherein a width (w) of the fluid pathway (602) is less than 5 mm, notably between 0.5 mm and 3 mm.

9. The test strip (600) according to any of claims 1 to 8, wherein the fluid pathway (602) comprises:

   - a sample pad (1110) configured to receive the fluid sample; and
   - a single wicking pad (1120), configured to absorb fluid from the fluid sample on the sample pad (1110),

   so that fluid flows from the sample pad (1110) to the single wicking pad (1120).

10. The test strip (600) according to claim 9, wherein the fluid pathway (602) further comprises:

    - a conjugate pad (1130) between the sample pad (1110) and the wicking pad (1120),
    - a reaction membrane (1140) between the conjugate pad (1130) and the wicking pad (1120).

11. The test strip (600) according to any of claims 1 to 10, wherein the analyte of interest is chosen between:

    - an ion of interest,
    - an antigen of interest,
    - an antibody of interest.

12. A cartridge (202) for an optical fluid analysis device (100), the cartridge (202) comprising at least one chamber (310) wherein the at least one chamber (310) comprises at least a test strip (600) according to any of claims 1 to 11.

13. The cartridge (202) according to claim 12, wherein at least one chamber (310) has a maximum dimension, e.g., a height (H), which is less than 10% more than the maximal expanse (E) of the test strip (600).

14. A fluid optical analysis device (100) comprising:

- a cartridge (202) according to claims 12 or 13,
- a station (200), configured to be positioned on a wall of a toilet bowl (102), the station (200) comprising:

+ a case (204) comprising a compartment (212) in which the cartridge (202) is at least partially received,
+ an injector, configured to inject fluid on the test strip (600),
+ an analyzer (400) to analyze a change of property of the test strip (600) due to fluid from the fluid sample.

15. The fluid optical analysis (100) device according to claim 14, wherein the analyzer (400) includes a light source (402, 404) and a light sensor, configured to emit and receive light.

**FIG. 1**

**FIG. 2**

FIG. 3

**FIG. 4**

(a)

A    B    α

(b)

B    α    A

(c)

B    α    A

# FIG. 5

600

606

(a)

E

602

F606

608

w

604

F604

606

(b)

E

F606

608

604

F604

600

Y

Z

X

**FIG. 6**

FIG. 7

FIG. 8

600

(a)

E

F    602

604

Z
Y    X

610

E

(b)

606

F

608

Y
Z    X

FIG. 9

**FIG. 10**

600

E

L

602

Y

Z

X

**FIG. 11**

**FIG. 12**

602

(a)

1210    1250  1260

N

A    D

(b)

P    S

C

(c)

C    P    D

1230    1240    C    P    D    1220

**FIG. 13**

FIG. 14

**FIG. 15**

FIG. 16

**Europäisches Patentamt**
**European Patent Office**
**Office européen des brevets**

# EUROPEAN SEARCH REPORT

**Application Number**

EP 23 18 0131

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 561 512 A1 (MAK WING CHEUNG [SE]; FILIPPINI D [SE]) 30 October 2019 (2019-10-30) * the whole document, in particular figures 11-15; paragraphs [0023], [0024], [0095], [0108]; claims 12-15 * | 1-13 | INV. G01N33/543 A61B10/00 |
| X | WO 00/77524 A1 (PRAXSYS BIOSYSTEMS INC [US]; THAYER R M [US] ET AL) 21 December 2000 (2000-12-21) | 1,3,4, 7-13 | |
| Y | * the whole document, in particular page 14, lines 17-24; claims 7, 19, 21; figures 4, 5 * | 14,15 | |
| X | US 2007/042427 A1 (GERDES J [US] ET AL) 22 February 2007 (2007-02-22) | 1-8, 11-13 | |
| Y | * the whole document, in particular figures 1-3; paragraph [0041] * | 14,15 | |
| X | WO 2016/193981 A1 (GIVEN IMAGING LTD [IL]) 8 December 2016 (2016-12-08) | 1,3-13 | TECHNICAL FIELDS SEARCHED (IPC) G01N A61B |
| Y | * the whole document, in particular page 19, line 18 to page 21, line 28; figures 2, 5a, 7a * | 14,15 | |
| Y | US 2023/105892 A1 (BARBEDETTE THIBAUT [FR] ET AL) 6 April 2023 (2023-04-06) * the whole document, in particular paragraphs [0011], [0060]; figures 1-14 * | 14,15 | |

The present search report has been drawn up for all claims

| Place of search | Date of completion of the search | Examiner |
|---|---|---|
| Munich | 15 January 2024 | Weber, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 18 0131**

This annex lists the patent family members relating to the patent documents cited in  the above-mentioned European search report.
The members are as contained in the European Patent Office EDP file on
The European Patent Office is in no way liable for these particulars which are merely given for the purpose of information.

15-01-2024

| Patent document cited in search report | | | Publication date | Patent family member(s) | | Publication date |
|---|---|---|---|---|---|---|
| EP | 3561512 | A1 | 30-10-2019 | EP | 3561512 A1 | 30-10-2019 |
| | | | | WO | 2019206632 A1 | 31-10-2019 |
| WO | 0077524 | A1 | 21-12-2000 | AT | E279726 T1 | 15-10-2004 |
| | | | | AU | 1628100 A | 02-01-2001 |
| | | | | CA | 2375453 A1 | 21-12-2000 |
| | | | | CN | 1277357 A | 20-12-2000 |
| | | | | DE | 69921199 T2 | 09-02-2006 |
| | | | | EP | 1185870 A1 | 13-03-2002 |
| | | | | ES | 2229797 T3 | 16-04-2005 |
| | | | | US | 6528323 B1 | 04-03-2003 |
| | | | | US | 2003157729 A1 | 21-08-2003 |
| | | | | WO | 0077524 A1 | 21-12-2000 |
| US | 2007042427 | A1 | 22-02-2007 | US | 2007042427 A1 | 22-02-2007 |
| | | | | WO | 2006130299 A2 | 07-12-2006 |
| WO | 2016193981 | A1 | 08-12-2016 | CN | 107683107 A | 09-02-2018 |
| | | | | EP | 3302266 A1 | 11-04-2018 |
| | | | | US | 2018160950 A1 | 14-06-2018 |
| | | | | WO | 2016193981 A1 | 08-12-2016 |
| US | 2023105892 | A1 | 06-04-2023 | CN | 115379804 A | 22-11-2022 |
| | | | | EP | 4087496 A2 | 16-11-2022 |
| | | | | US | 2023105892 A1 | 06-04-2023 |

EPO FORM P0459

For more details about this annex : see Official Journal of the European Patent Office, No. 12/82

**EP 4 481 387 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021175909 A
- WO 2021175944 A
- WO 2023036805 A
- WO 2023036806 A
- WO 2023036808 A
- WO 2023036809 A